# EUROPEAN PATENT APPLICATION

(11) **EP 2 047 816 A2**
(43) Date of publication of application: **15.04.2009**
(21) Application number: 08253241.7
(22) Date of filing: 06.10.2008
(51) Int. Cl.: A61B 18/12

(54) **Electrosurgical system**

(30) Priority: 09.10.2007 GB 0719733
(71) Applicant: GYRUS MEDICAL LIMITED, Cardiff CF3 0LT (GB)
(72) Inventor: Newton, Michael David, Newport NP10 8JE (GB)
(74) Representative: Blatchford, William Michael

(57) **Abstract**

An electrosurgical system includes a generator (10) for generating radio frequency power, an electrosurgical instrument (12) including at least first and second electrodes (3A, 3B) carried on the instrument, and a monopolar patient return electrode (11) separate from the instrument. The generator comprises at least one source (1) of radio frequency (RF) power, and has a first supply state in which the RF waveform is supplied between the first electrode (3A) and the patient return electrode (11), and a second supply state in which the RF waveform is supplied between a second electrode (3B) and the monopolar patient return electrode (11). A controller is operable to control the generator (10) such that, in at least one mode of the generator, feeding means within the generator is adapted to alternate between the first and second supply states.

## Description

This invention relates to an electrosurgical system including a monopolar electrosurgical instrument for use in the treatment of tissue.

Both monopolar and bipolar electrosurgery are well-established techniques. In monopolar electrosurgery, an electrosurgical instrument has a single electrode and a patient return plate is attached to the patient well away from the electrosurgical instrument. The electrosurgical current flows from the electrode through the patient to the return plate.

In bipolar electrosurgery, the electrosurgical instrument includes spaced first and second electrodes, and there is no patient return plate. The current flows from one electrode through the patient to the other, and so the current flow is kept to a much more localised area.

Both monopolar and bipolar electrosurgery are known to have certain advantages and disadvantages. Monopolar electrosurgery is known to produce very effective tissue coagulation, but there is always the danger of stray current paths causing the unwanted treatment of tissue spaced from the monopolar electrode. Bums to the patient in the area of the return plate have also been known. Bipolar electrosurgery is generally considered to be a safer option, as the current is constrained within a smaller area, but it is sometimes difficult to obtain as penetrative a coagulation effect with a bipolar instrument.

For this reason perhaps, there have been previous attempts to provide the option of either monopolar or bipolar electrosurgery from a single generator. It is well known to allow both a monopolar and a bipolar instrument to be connected to the generator, with some form of switch to select which one of the instruments is to be activated at any one time, see examples include US patents 4,171,700, 4,244,371, 4,559,943, 5,951,545 and 6,113,596. US patent 5,472,442 is different in that a single instrument can be used in either a monopolar or bipolar mode, but once again a choice must be made as to which one of monopolar or bipolar modes is to be activated at any one time.

The present applicant describes in GB patent application 0708783.6 and in US patent application 60/924961 an electrosurgical system in which a blended signal consisting of a monopolar component and a bipolar component can be supplied to tissue from a single instrument. This invention seeks to provide an enhancement or alternative to such a system, particularly when the instrument is being used in a monopolar mode.

According to a first aspect of the invention, an electrosurgical system includes a generator for generating radio frequency power, and an electrosurgical instrument including at least first and second electrodes carried on the instrument, the first and second electrodes being shaped so as to contact tissue without penetrating it, and a monopolar patient return electrode separate from the instrument, the generator comprising at least one source of radio frequency (RF) power, and having a first supply state in which an RF waveform is supplied between a first electrode of the electrosurgical instrument and the monopolar return electrode, and a second supply state in which an RF waveform is supplied between the second electrode and the monopolar patient return electrode, and feeding means operable such that, in at least one mode of the generator, the feeding means is adapted to alternate between the first and second supply states.

The monopolar patient return electrode is described as being separate from the instrument. This is to say that the monopolar patient return electrode is designed to be attached to the patient at a location remote from the area where the instrument is in contact with the patient. Conceivably, the patient return electrode could still be supplied together with the electrosurgical instrument, and may even be physically attached thereto, for example by means of a long cord or tie. The description of the monopolar patient return electrode as being "separate" refers to its remote location on the patient, as opposed to any lack of connection with the electrosurgical instrument.

The invention has particular advantages when the electrosurgical instrument is such that the first and second electrodes are provided on opposite faces of the electrosurgical instrument. Consider an arrangement in which such an instrument is used adjacent tissue in its monopolar mode, with both the first and second electrodes being energized. The monopolar patient return plate may well be attached to the patient in a location in which the current path between the first electrode and the patient return plate is much shorter or otherwise offers a much lower resistance as compared with the current path between the second electrode and the patient return plate. In this circumstance, the tissue adjacent the first electrode may be effectively coagulated, but there may be less coagulation (or even little or no coagulation) adjacent the second electrode.

With the feeding means adapted to alternate between the first and second supply states to produce an alternating signal, the monopolar signal alternates between a first supply state (in which it is supplied between the first electrode and the patient return plate) and a second supply state (in which it is supplied between the second electrode and the patient return plate). Thus, there will be periods during the supply of the monopolar signal when it is supplied solely to the first electrode, and other periods when it is supplied solely to the second electrode. In this way, effective coagulation is promoted adjacent each electrode, and therefore on both sides of the electrosurgical instrument.

The first duty cycle is conveniently that part of the overall signal that is in the first supply state, and the second duty cycle is that part of the overall signal that is in the second supply state. Thus, the first and second duty cycles may be the proportions or percentages of time during periods of activation of the generator that RF power is delivered with the generator in the first and second supply states respectively. During periods of generator activation, RF power may be delivered to the instrument continuously or as a series of pulses. In one convenient arrangement, the feeding means operates such that the first and second duty cycles are both 50%. This effectively alternates the monopolar signal equally between the first and second electrodes.

Alternatively, there is provided adjustment means, operable by the user of the electrosurgical system, for changing at least one duty cycle. In this way, the monopolar signal can be supplied preferentially to one of the electrodes, or supplied preferentially to one electrode and then to the other. In a further alternative arrangement, the first and second duty cycles are such that there are periods between the first and second supply states during which the generator does not supply an RF waveform to either the first or second electrodes. For example, a first duty cycle of 30% and a second duty cycle of 50% would see a gap between the first and second supply states, in which the RF waveform was not supplied to either electrode, this gap representing 20% of the overall cycle. This gap may be present between the first and second supply states, or after the second supply state (and before the generator reverts to the first supply state). Alternatively, the gaps may be present between each of the supply states (i.e. following the first supply state and following the second supply state) as preferred. Additionally or alternatively, the first and second duty cycles are such that there are periods during which the generator supplies an RF waveform to both the first and second electrodes simultaneously.

In one conceivable arrangement, a mechanical cutting blade is provided between the two electrodes. In this way, if cutting is required, whether before or after coagulation by the first and second electrodes, the instrument is moved across the tissue to allow the cutting blade to transect the tissue. Alternatively, the cutting blade is constituted by an electrosurgical cutting blade, such that the electrosurgical instrument includes at least a third electrode, and the generator is adapted, in an alternative mode of operation, to supply a cutting RF waveform between the third electrode and one of the other electrodes. In a first arrangement, the other electrode is one or both of the first and second electrodes, such that the electrosurgical cutting is a bipolar cutting action. Alternatively, the other electrode is the monopolar patient return electrode, in which case the electrosurgical cutting is a monopolar cutting action between the third electrode and the remote patient plate. Conceivably, the cutting action could be a blend of both bipolar and monopolar cutting, as described in GB patent application 0708783.6 and in US patent application 60/924961, the contents of which are hereby incorporated by reference.

The electrosurgical system conceivably includes means for measuring a parameter associated with the electrosurgical procedure, the controller adjusting at least one duty cycle automatically in response to the measured parameter. In this way, the electrosurgical system adjusts itself dynamically in response to different operating conditions, selecting greater or lesser proportions of the first and second supply states, as required for effective operation. Conveniently, the measured parameter is the impedance measured between each of the first and second electrodes and the patient return electrode. Thus, when the measured impedance is low for one of the first or second electrodes, indicating a relatively fluid surgical environment associated with bleeding tissue, the electrosurgical system could increase the duty cycle associated with that electrode to provide additional coagulating power. Conversely, when the measured impedance is higher, indicating a relatively dry surgical environment, the electrosurgical system could decrease the duty cycle associated with that electrode.

In another convenient arrangement, the feeding means operates such that at least one duty cycle varies according to a predetermined progression. This provides a dynamically changing electrosurgical signal, without the user selecting different operating settings, or the system performing dynamic measurement of operating parameters. For example, experience could show that the most effective tissue coagulating waveform for a particular tissue or vessel type is a particular combination of the first and second supply states, changing over time. This could be preprogrammed into the electrosurgical generator, such that it is automatically performed without the need for any additional intervention from the user. Different predetermined progressions of duty cycle may be appropriate for different types of tissue, or for different surgical procedures, as will be readily established by users of the electrosurgical system.

According to another aspect of the invention, there is provided an electrosurgical system electrosurgical system including a generator for generating radio frequency (RF) power, an electrosurgical instrument including at least first and second tissue surface treatment electrodes carried on the instrument and each shaped to apply an electrosurgical RF voltage to a tissue surface position, and a monopolar patient return electrode separate from the instrument for application to a different part of the patient's body, the generator comprising at least one source of RF power and first, second and third output connections coupled to the first second and return electrodes respectively, and having a first supply state in which an RF waveform is supplied via the first and third output connections between a first electrode of the electrosurgical instrument and the monopolar return electrode, and a second supply state in which an RF waveform is supplied via the second and third output connections between the second electrode and the monopolar patient return electrode, and feeding means operable such that, in at least one mode of the generator, the feeding means is adapted to alternate between the first and second supply states.

It is preferred that the instrument has a unitary electrode assembly comprising at least the first and second electrodes and an electrically insulative member located between the first and second electrodes. The electrode assembly may be shaped to allow both the first and the second electrode to contact the tissue surface portion referred to above simultaneously whereby, in use of the system, electrosurgical RF currents are caused to flow through tissue to be treated from the contacted tissue surface portion to the return electrode selectively via the first electrode and the second electrode such that tissue coagulation occurs from the surface portion downwardly into the tissue. Each of the first and second electrode has a transversely extending end portion for contacting the tissue surface portion, each of the electrodes being arranged generally longitudinally of the instrument.

The invention will now be described in more detail, by way of example only, with reference to the accompanying drawings, in which;
Figure 1 is a schematic sectional view of an electrosurgical system according to the invention;
Figure 2 is a schematic perspective view of an electrosurgical instrument useable as part of the system of Figure 1;
Figure 3, 7, 8 and 10 are schematic cross-sectional views showing the effect on tissue of different modes of operation of the electrosurgical instrument of Figure 2;
Figures 4, 5, 6 and 9 are schematic diagrams showing the electrosurgical system of Figure 1 in different modes of operation;
Figures 11a to 11e are schematic diagrams showing different outputs of the electrosurgical system of Figure 1; and
Figures 12 to 15 are schematic diagrams showing an alternative electrosurgical system in different modes of operation.

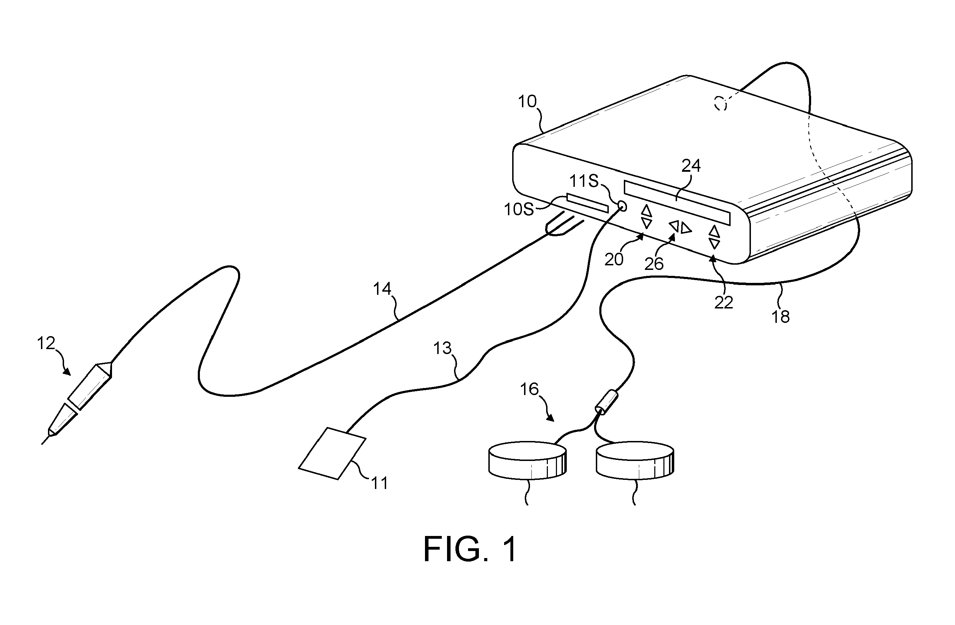

Referring to Figure 1, a generator 10 has an output socket 10S providing a radio frequency (RF) output for an instrument 12 via a connection cord 14. An output socket 11S provides a connection for a patient return plate 11, via cord 13. Activation of the generator may be performed from the instrument 12 via a control connection in cord 14 or by means of a footswitch unit 16, as shown, connected separately to the rear of the generator 10 by a footswitch connection cord 18. In the illustrated embodiment, footswitch unit 16 has two footswitches 16A and 18B for selecting a coagulation mode and a cutting mode of the generator respectively. The generator front panel has push buttons 20 and 22 for respectively setting coagulation and cutting power levels, which are indicated in a display 24. Push buttons 26 are provided as an alternative means for selection between coagulation and cutting modes.

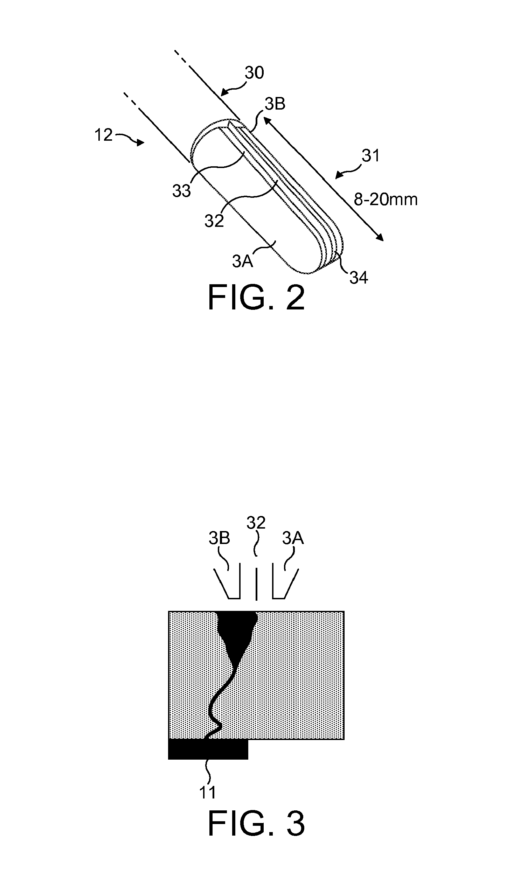

Figure 2 shows a typical design for the electrosurgical instrument 12. The instrument 12 comprises an instrument shaft 30 at the distal end of which is a unitary electrode assembly shown generally at 31. The electrode assembly 31 extends generally longitudinally of the instrument and comprises a central cutting blade 32 disposed between two larger coagulation electrodes 3A and 3B. The cutting blade 32 may be a mechanical cutting blade, or an electrosurgical cutting electrode. Where the blade is a cutting electrode, insulating layer 33 separates the cutting electrode 32 from the first coagulating electrode 3A while insulating layer 34 separates the cutting electrode from the second coagulating electrode 3B. The cutting blade 32 protrudes slightly beyond the two coagulating electrodes. As seen in Figure 2, both the cutting blade and the coagulating electrodes each has a transversely extending portion at the end of the electrode assembly and longitudinally extending side portions.

When the user intends the instrument to cut tissue, the generator applies a cutting RF waveform between the cutting electrode 32 and one or both of the coagulating electrodes 3A and 3B. The protruding nature of the cutting electrode 32 helps to provide a cutting action when the electrode 32 is brought into contact with tissue. When the user intends the instrument to coagulate tissue, the electrosurgical generator supplies an RF waveform between the electrodes 3A and 3B as well as the patient return plate (not shown in Figure 2). This coagulation of the tissue will now be described in more detail.

Figure 3 shows the coagulating action of an instrument such as that shown in Figure 2. The coagulating electrodes are shown at 3A and 3B, and the cutting blade at 32. The patient return plate 11 is affixed to the tissue at a site remote from the instrument 12, and both electrodes 3A and 3B are supplied with a coagulating RF waveform. Due to the positioning of the return plate 11, the current path from the electrode 3B to the return plate 11 is shorter than that between the electrode 3A and the return plate. As a result, the coagulating action of the instrument 12 is primarily adjacent electrode 3B, with little or no coagulation produced adjacent electrode 3A.

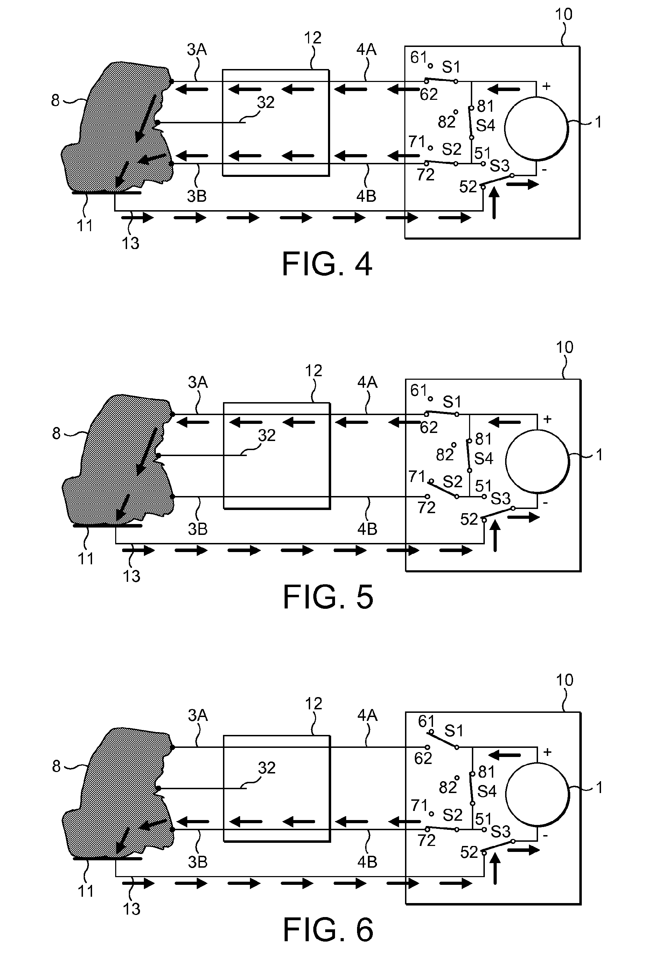

Figure 4 shows the electrosurgical system corresponding to this arrangement. Generator 10 has an RF power source 1. Instrument 12 includes the electrodes 3A and 3B, and power source 1 is connected between electrodes 3A and 3B via lines 4A and 4B. The generator is also connected to the patient return plate 11 (via cord 13). A first switch S1 governs the connection between the RF power source and the electrode 3A (via line 4A). Switch S1 has a first position 62 in which the power source is connected to the electrode 3A, and a second position 61 in which the power source is not connected to the electrode 3A. Similarly, a second switch S2 governs the connection between the RF power source and the electrode 4A (via line 4B). Switch S2 has a first position 72 in which the power source is connected to the electrode 4A, and a second position 71 in which the power source is not connected to the electrode 4A. There is also a third switch S3, governing the connection between the RF power source and the patient return plate 11 (via line 13). A fourth switch S4 allows the power source 1 to be connected to both first and the second switches S1, S2 when in its first position 81.

Switch S3 has a first position 52 in which the patient plate 11 is connected to the source, and a second position 51 in which the patient plate 11 is not connected to the source. Switches S1, S2, S3 and S4 are high-speed transistor switches, capable of switching between two alternate positions many times per second.

In the arrangement of Figure 4, the switches S1, S2 and S4 are in their first positions, 62, 72 and 81 respectively, connecting the power source 1 to both electrodes 3A and 3B simultaneously. Switch S3 is also in its first position 52, connecting the patient return plate 11 to the generator. This is the configuration shown in Figure 3, in which both electrodes are energized, but preferential coagulation may occur due to the location of the patient return plate 11.

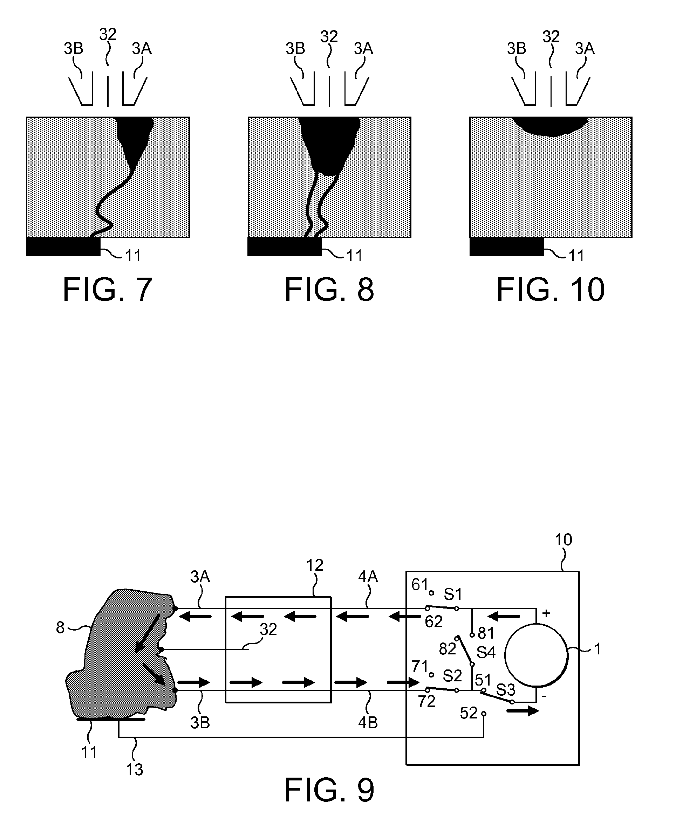

In order to overcome this preferential coagulation, the first and second switches S 1 and S2 are operated in tandem and independently as shown in Figures 5 and 6. Switch S3 remains in its first position 52, connecting the patient return plate 11 to the generator and switch S4 remains in its first position 81 interconnecting switches S1 and S2. Figure 5 shows the situation when switch S1 is in its first position 62 and switch S2 is in its second position 71. In this arrangement the power source 1 is connected to electrode 3A but disconnected from electrode 3B. This constitutes a first supply state in which the RF waveform is supplied to the first 3A of the two electrodes. Figure 6 shows the opposite situation when switch S1 is in its second position 61 and switch S2 is in its first position 72. In this arrangement the power source 1 is connected to the second electrode 3B and disconnected from the first electrode 3A. This constitutes a second supply state in which the RF waveform is supplied to the second of the two electrodes. Figure 7 illustrates current flow in the tissue in the first supply state, shown in Figure 5. In this situation, the coagulating current is forced to flow from electrode 3A and cause coagulation adjacent this electrode, even though the current path to the patient return plate 11 is longer than that from electrode 3B. This shorter current path is temporarily unavailable, as electrode 3B is disconnected from the power source 1. In the second supply state, shown in Figure 6, current flow in the tissue follows the shorter path, as illustrated in Figure 3.

The switches alternate in tandem between these two positions at a frequency of between 5 and 100Hz to provide a continuous rapid alternation between the first and second electrodes. Thus the tissue effect achieved in the tissue 8 in the region of the electrodes 3A and 3B is a combination of the tissue effects shown in Figures 3 and 7, with a more even tissue coagulation than would be achieved by energizing both electrodes simultaneously. This is illustrated in Figure 8.

The system of Figures 4 to 6 can also be operated in bipolar coagulation mode, as illustrated in Figure 9. In this arrangement switch S4 is in a second position 82 so that it is open, and switches S 1 and S2 are both placed in their first positions, 62 and 72 respectively, connecting the power source between the first and second electrodes 3A and 3B. Switch S3 however is placed in its second position 51, isolating the patient return plate from the power source 1. Since switch S4 is open, bipolar operation is allowed, the coagulating RF waveform being supplied between the first and second electrodes 3A and 3B in a bipolar manner, causing a more localized coagulating effect on the tissue adjacent the electrodes, as illustrated in Figure 10. Summarising, switch S4 is set closed or open for monopolar or bipolar operation respectively. In the monopolar mode, the first and second switches 51, 52 control the distribution and feeding of current between the first and second electrodes 3A, 3B.

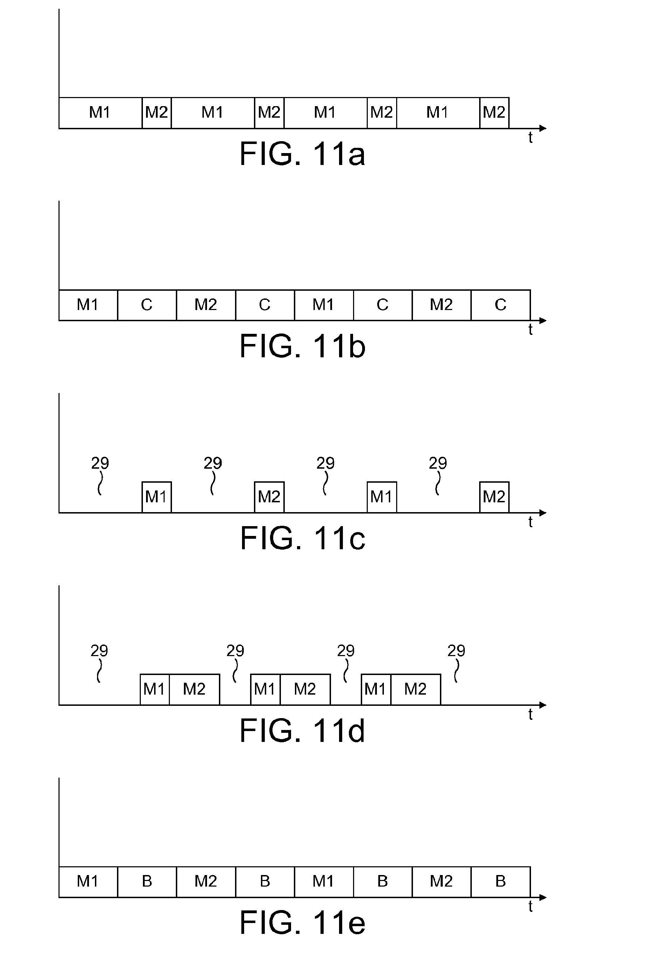

Figures 11 a to 11e show different arrangements for the timings for the switches S 1 and S2. In the figures, the switches are in the positions shown in Figure 5 for the periods shown as marked with "M1", indicating the energizing of the first electrode 3A. Conversely, the switches are in the positions shown in Figure 6 for the periods shown as marked with an "M2", indicating the energizing of the second electrode 3B. In Figure 11a, the first supply state of Figure 5 is activated for approx 75% of the duty cycle, and the second supply state of Figure 6 is activated for the remaining 25%). Thus the tissue effect will be much more influenced by the electrode 3A than that of electrode 3B.

In Figure 11b the first and second duty cycles are both 33%, with energy being delivered alternately to electrodes 3A and 3B in the periods M1 and M2. Between these two periods, the switches S1 and S2 are in the position shown in Figure 4, with both electrodes 3A and 3B being energized. These periods are marked with "C" to show the combined activation of the electrodes.

In Figure 11a the switches S 1 and S2 operate in unison, so that the second electrode takes over from the first electrode without an interruption, and vice versa. Thus the coagulating signals are supplied consecutively to the tissue 8, without a break. In Figures 11c and 11d a deliberate time gap 29 is left between the signals. Referring to Figure 11c, a gap 29 is left after between the activation of each electrode, with both switches S1 and S2 remaining in their second (open) positions for a period before the next switch closes. In Figure 11d, there is a continuous transition from the first supply state M1 to the second supply state M2, but a gap 29 is left after between the second supply state M2 before the system returns to the first supply state M1. Clearly, with the gaps of Figures 11c and 11d, the first and second duty cycles do not total 100%. In Figure 11c, the first and second duty cycles are both approx 20%, (meaning that the gap 29 constitutes 60% of the overall cycle time). In Figure 11d, the first duty cycle is still 20% and the second is approx 50% (meaning that the gap 29 constitutes 30% of the overall cycle time).

In Figure 11e the first and second duty cycles are both 33%, with energy being delivered alternately to electrodes 3A and 3B in the periods M1 and M2. Between these two periods, the switch S3 is in the position shown in Figure 9, with both electrodes 3A and 3B being energized in a bipolar mode. These periods are marked with "B" to show the bipolar activation of the electrodes. This combination of monopolar and bipolar activation is described in more detail in GB patent application 0708783.6 and in US patent application 60/924961, as previously mentioned.

In Figures 11 a to 11e the duty cycle is constant for one time period as compared with another. However, this does not necessarily need to be the case, as the system may be operated such that first and second duty cycles vary with time. These arrangements are also described in more detail in GB patent application 0708783.6 and in US patent application 60/924961. These applications also describe how any duty cycle can also be adaptively controlled based on a parameter associated with the electrosurgical procedure, such as the tissue impedance. If the electrosurgical system detects a low tissue impedance associated with the first electrode 3A (indicating a relatively fluid surgical environment associated with bleeding tissue), the first duty cycle would be adjusted upwardly to increase the proportion of monopolar signal applied to the tissue by the electrode 3A. Conversely, if the electrosurgical system detects a relatively low tissue impedance associated with the second electrode 3B (indicating a relatively fluid surgical environment adjacent the electrode 3B), the second duty cycle would be adjusted upwardly to increase the proportion of monopolar signal applied to the tissue by the electrode 3B. Thus the electrosurgical system can adapt automatically to changes in the surgical environment, without the need for a manual adjustment of the generator by the surgeon.

The central cutting electrode 32 has been depicted in Figures 4 to 10 as being merely a mechanical cutting blade, with no electrical connection to the generator 10. However, as described in GB patent application 0708783.6 and in US patent application 60/924961, the cutting blade may also be connected to the generator to constitute an electrosurgical cutting electrode, without departing from the scope of the present invention.

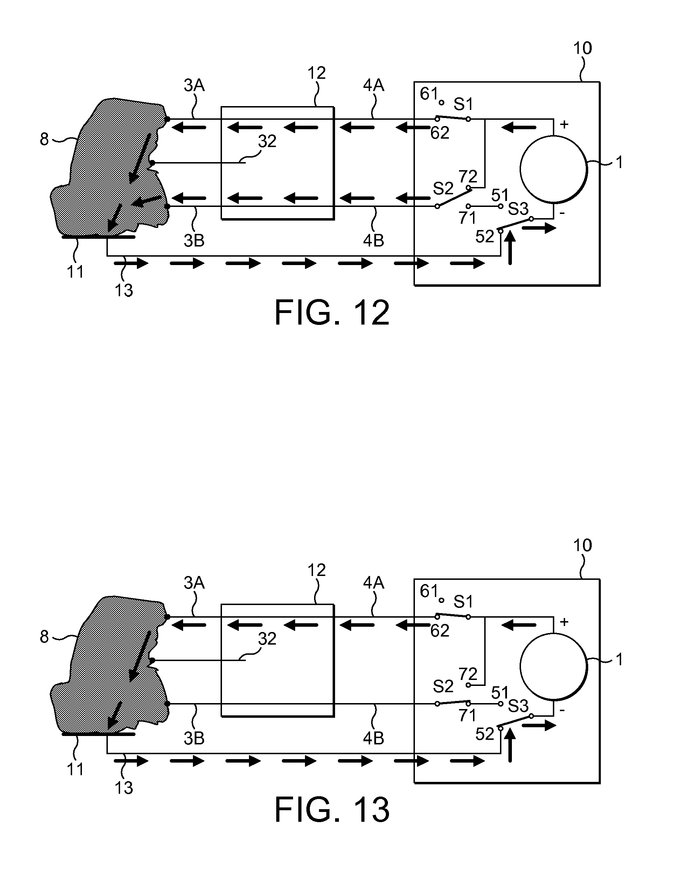

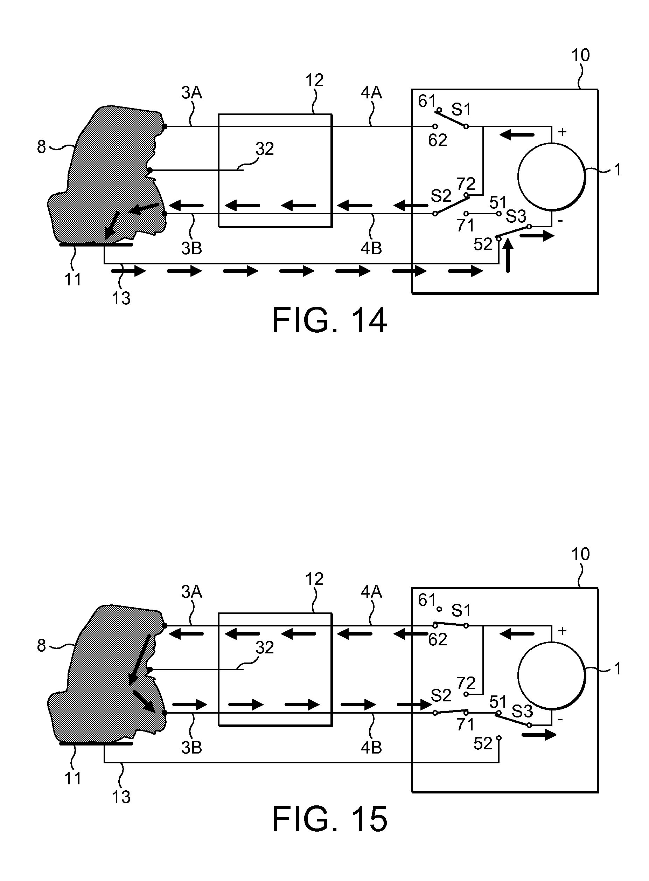

Referring to Figures 12 to 15, in an alternative system in accordance with the invention, the four switches S1 - S4 of the first system described above with reference to Figures 4, 5, 6 and 9 are replaced with three switches S1, S2, S3, switch S2 being a two-way switch in a reversed configuration compared with switch S2 in the first embodiment so that the second coagulating electrode 3B is connected to either one or the other of the output terminals of the RF power source 1. With switch S 1 closed and switch S2 in its first position 72, both electrodes 3A, 3B are fed from one terminal of the power source 1 to produce the same effect as the arrangement of Figure 4. Referring to Figure 13, when switch S 1 is closed, but switch S2 is in its second position 71, only the first electrode 3A is operative, the system being in the above-mentioned first supply state. Conversely, referring to Figure 14, when the first switch S1 is open (position 61) and S2 is in its first position 72, only the second electrode 3B is energized and the system is in the above-mentioned second supply state described above with reference to Figure 6. Operation of the switches S 1 and S2 in tandem and independently between their positions shown in Figure 13 and their positions shown in Figure 14 according to predetermined duty cycles allows a combination of tissue effects to be achieved, as described above with reference to Figures 3, 7 and 8.

For bipolar operation, switch S 1 is in its closed position, switch S2 is in its second position 71, and switch S3 is in its second position 51, as illustrated in Figure 15.

Those skilled in the art will appreciate that variations on the precise examples given herein can be made without departing from the scope of the present invention. For example, a range of different arrangements for varying the duty cycle, in addition to those described herein, could be readily derived depending on the tissue to be treated, the surgical procedure under consideration, or even the particular preference of each individual surgeon. As previously mentioned, any of the embodiments discussed herein can be employed with or without an additional cutting electrode, or in combination with other waveforms such as blended cut and coagulation, blended monopolar and bipolar, or both.

## Claims

1. An electrosurgical system including a generator (10) for generating radio frequency power, an electrosurgical instrument (12) including at least first and second electrodes (3A, 3B) carried on the instrument, the first and second electrodes (3A, 3B) being shaped so as to contact tissue without penetrating it, and a monopolar patient return electrode (11) separate from the instrument,
the generator (10) comprising at least one source (1) of radio frequency (RF) power, and having a first supply state in which an RF waveform is supplied between the first electrode (3A) of the electrosurgical instrument (12) and the monopolar return electrode (11), and a second supply state in which an RF waveform is supplied between the second electrode (3B) and the monopolar return electrode (11), and feeding means operable such that, in at least one mode of the generator, the feeding means is adapted to alternate between the first and second supply states.

2. An electrosurgical system according to claim 1, wherein the first duty cycle is that part of the overall signal that is in the first supply state, and the second duty cycle is that part of the overall signal that is in the second supply state.

3. An electrosurgical system according to claim 2, wherein the feeding means operates such that the first and second duty cycles are both 50%.

4. An electrosurgical system according to claim 2, wherein there is provided adjustment means, operable by the user of the electrosurgical system, for changing at least one duty cycle.

5. An electrosurgical system according to claim 2, wherein the first and second duty cycles are such that there are periods between the first and second supply states during which the generator (10) does not supply an RF waveform to either the first or second electrodes (3A, 3B).

6. An electrosurgical system according to claim 2, wherein the first and second duty cycles are such that there are periods during which the generator (10) supplies an RF waveform to both the first and second electrodes (3A, 3B) simultaneously.

7. An electrosurgical system according to any preceding claim, wherein the first and second electrodes (3A, 3B) are provided on opposite faces of the electrosurgical instrument (12).

8. An electrosurgical system according to claim 7, wherein a mechanical cutting blade (32) is provided between the two electrodes (3A. 3B).

9. An electrosurgical system according to any of claims 1 to 7, wherein the electrosurgical instrument includes at least a third electrode (32), and the generator (10) is adapted, in an alternative mode of operation, to supply a cutting RF waveform between the third electrode and one of the other electrodes (3A, 3B).

10. An electrosurgical system according to claim 9, wherein the other electrode is one or both of the first and second electrodes (3A, 3B).

11. An electrosurgical system according to claim 9, wherein the other electrode is the monopolar patient return electrode (11).

12. An electrosurgical system including a generator (10) for generating radio frequency (RF) power, an electrosurgical instrument (12) including at least first and second tissue surface treatment electrodes (3A, 3B) carried on the instrument and each shaped to apply an electrosurgical RF voltage to a tissue surface position, and a monopolar patient return electrode (11) separate from the instrument for application to a different part of the patient's body,
the generator (10) comprising at least one source (1) of RF power and first, second and third output connections coupled to the first second and return electrodes respectively, and having a first supply state in which an RF waveform is supplied via the first and third output connections between a first electrode (3A) of the electrosurgical instrument and the monopolar return electrode (11), and a second supply state in which an RF waveform is supplied via the second and third output connections between the second electrode (3B) and the monopolar patient return electrode (11), and feeding means operable such that, in at least one mode of the generator, the feeding means is adapted to alternate between the first and second supply states.

13. A system according to claim 12, wherein the instrument (12) has a unitary assembly (31) comprising at least the first and the second electrodes (3A, 3B) and an electrically insulative member (33, 34) located between the first and second electrodes, the electrode assembly being shaped to allow both the first and the second electrode to contact the said tissue surface portion simultaneously whereby, in use of the system, electrosurgical RF currents are caused to flow through tissue to be treated from the contacted tissue surface portion to the return electrode (11) selectively via the first electrode (3A) and the second electrode (3B) such that tissue coagulation occurs from the surface portion downwardly into the tissue.

14. A system according to claim 12 and claim 13, wherein each of the first and second electrodes (3A, 3B) has a transversely extending end portion for contacting the said tissue surface portion.
